# FASCICULE DE BREVET EUROPEEN

(11) **EP 3 806 628 B1**
(45) Date de publication et mention de la délivrance du brevet: **07.08.2024**
(21) Numéro de dépôt: 19790595.3
(22) Date de dépôt: 11.06.2019
(51) Int. Cl.: A01K 67/027, A61F 13/00

(54) **MODELE POUR L'EVALUATION DE TRAITEMENTS POUR LA CICATRISATION DES ULCERES DE PRESSION**
MODELL ZUR AUSWERTUNG VON BEHANDLUNGEN ZUR HEILUNG VON DRUCKGESCHWÜREN
MODEL FOR EVALUATING TREATMENTS FOR THE HEALING OF PRESSURE ULCERS

(30) Priorité: 12.06.2018 FR 1855141
(43) Date de publication de la demande: 21.04.2021
(73) Titulaire: URGO RECHERCHE INNOVATION ET DEVELOPPEMENT, 21300 Chenôve (FR); Centre National de la Recherche Scientifique (CNRS), 75016 Paris (FR); UNIVERSITE CLAUDE BERNARD - LYON 1, 69100 Villeurbanne (FR)
(72) Inventeur: REMOUE, Noëlle, 69007 Lyon (FR); SIGAUDO-ROUSSEL, Dominique Béatrice Jeanne, 69003 Lyon (FR)
(74) Mandataire: Plasseraud IP
(86) Numéro de dépôt international: PCT/FR2019/051398
(87) Numéro de publication internationale: WO 2019/239051

(56) Documents cités:
- US-A1- 2014 370 078
- "Pressure Ulcer Research", 2005, SPRINGER-VERLAG, Berlin/Heidelberg, ISBN: 978-3-540-25030-2, article ANKE STEKELENBURG ET AL: "Compression-Induced Tissue Damage: Animal Models", pages: 187 - 204, XP055573032, DOI: 10.1007/3-540-28804-X_12
- YURIKO TAKEUCHI ET AL: "Development of Novel Mouse Model of Ulcers Induced by Implantation of Magnets", SCIENTIFIC REPORTS, vol. 7, no. 1, 7 July 2017 (2017-07-07), XP055572289, DOI: 10.1038/s41598-017-05250-y
- ISTVAN STADLER, REN-YU ZHANG, PHILLIP OSKOUI, MEGAN BS S. WHITTAKER & RAYMOND J. LANZAFAME: "DEVELOPMENT OF A SIMPLE, NONINVASIVE, CLINICALLY RELEVANT MODEL OF PRESSURE ULCERS IN THE MOUSE", JOURNAL OF INVESTIGATIVE SURGERY, vol. 17, no. 4, 2004, pages 221 - 227, XP009512094, DOI: 10.1080/08941930490472046
- ANONYMOUS: "Shear: a contributory factor in pressure ulceration", 27 May 2016 (2016-05-27), XP055572878, Retrieved from the Internet <URL:https://web.archive.org/web/20160527133650/https://www.npuap.org/wp-content/uploads/2012/03/Shear_slides_with_animation.pdf> [retrieved on 20190321]
- METIN YAVUZ ET AL: "Plantar Shear Stress in Individuals With a History of Diabetic Foot Ulcer: An Emerging Predictive Marker for Foot Ulceration", DIABETES CARE, vol. 40, no. 2, 29 November 2016 (2016-11-29), US, pages e14 - e15, XP055573126, ISSN: 0149-5992, DOI: 10.2337/dc16-2204
- ANONYMOUS: "Optiskin Film , Pansements stériles - URGO", 22 March 2017 (2017-03-22), XP055573164, Retrieved from the Internet <URL:https://web.archive.org/web/20170322114240/http://www.urgo.fr/film-optiskin/> [retrieved on 20190322]
- DINSDALE: "Decubitus ulcers in swine: light and electron microscopy study of pathogenesis.", ARCH PHYS MED REHABIL., vol. 54, no. 2, February 1973 (1973-02-01), pages 51 - 56, XP055572784

## Description

### DOMAINE TECHNIQUE

La présente invention se rapporte à une méthode d'évaluation d'un traitement(s) pour la cicatrisation des ulcères de pression. De préférence, cette évaluation permet un suivi de la cicatrisation des ulcères de pression chez les rongeurs présentant un diabète, en particulier chez les rongeurs présentant un diabète neuropathique ou neuro-ischémique.

Cette évaluation permet de suivre des paramètres de cicatrisation tels que l'inflammation, la granulation, l' épithélialisation. La présente invention se rapporte également à un modèle animal d'évaluation d'ulcère de pression.

### ARRIERE PLAN

L'ulcère de pression ou escarre est considéré comme un problème clinique majeur. Son développement est plus fréquent chez les patients diabétiques, les sujets avec perte de mobilité, dont les patients âgés hospitalisés et alités, ainsi que les patients avec l'altération de la moelle épinière (Allman 1989; Amlung, Miller et al. 2001). L'incidence de développement d'escarres est estimée à 15% des patients diabétiques (Reiber, Vileikyte et al. 1999) et 84% d'entre eux subiront une amputation des extrémités inférieures (Reiber, McDonell et al. 1995).

L'ulcère de pression se définit par un ensemble de lésions de la peau et/ou des tissus mous sous-jacents habituellement localisés sur une proéminence osseuse. Les zones de proéminences osseuses estimées comme zones à risque sont le sacrum, le talon, la tubérosité ischiatique et le coude (Amlung, Miller et al. 2001; Bansal, Scott et al. 2005).

En clinique, la sévérité des escarres est définie du stade I au stade IV. Le stade I définit une peau intacte qui apparait rouge dont un érythème qui ne disparait pas à la levée de la pression. Le stade II est caractérisé par des lésions impliquant l'épiderme et en partie le derme et se présente comme une ulcération superficielle. Le stade III est une lésion du tissu sous-cutané avec décollement. Le stade IV inclut les lésions s'étendant de la peau et des muscles sur les proéminences osseuses.

Il a été établi que la réponse hyperémique aux fortes pressions indiquait que la peau ne pouvait résister à l'application répétitive de fortes pressions, pendant la marche notamment (Newrick, Cochrane et al. 1988). Même si l'application de la pression prolongée est considérée comme le facteur principal des ulcères de pression, la pathologie de l'ulcération n'est pas entièrement explorée (Bouten, Oomens et al. 2003).

Il existe plusieurs hypothèses pour désigner les facteurs de risque d'apparition de lésions liées à la pression (Mak, Zhang et al. 2010; Boulton 2013). Cependant, l'apparition des blessures pourrait être liée à la concordance de plusieurs évènements néfastes au cours du diabète. Ainsi, on peut définir la tolérance du tissu à résister à la pression comme la capacité du tissu répondre à la pression sans développer d'ulcère de pression (Bergstrom, Horn et al. 2005).

Les neuropathies périphériques sensorielles (Reiber, Vileikyte et al. 1999; Boulton 2004) et les neuropathies du système nerveux moteur (Bus, Yang et al. 2002) peuvent inhiber la perception de la douleur et la sensation de toucher. Les changements précoces de la microcirculation cutanée et du métabolisme musculaire (Greenman, Panasyuk et al. 2005) peuvent induire une ischémie locale et l'hypoxie tissulaire résultant de l'occlusion du flux sanguin, ainsi que des changements structurels au niveau du tissu cutané, par exemple une diminution de la flexibilité du réseau de collagène initiée par la glycation des protéines observée au cours de l'hyperglycémie (Paul and Bailey 1996). L'accumulation de substances toxiques au niveau tissulaire est liée à un défaut du transport lymphatique au cours de la réduction de la perfusion, révélant l'importance de l'environnement pour la survie tissulaire. La nécrose tissulaire qui en résulte provoque l'ulcération (Bouten, Oomens et al. 2003).

Les changements structurels sont un facteur important pour le développement d'ulcères de pression. Il a été montré qu'au cours du vieillissement la diminution de l'élasticité et du tissu adipeux de la peau sèche, associée à la diminution de l'épaisseur de l'épiderme et derme contribuaient à l'apparition de lésions (Witkowski and Parish 2000; Garcia and Thomas 2006).

Les facteurs extrinsèques sont les infections, un traumatisme mineur, une déformation de la peau induisant une formation calleuse (Robertson, Mueller et al. 2002), un stress mécanique répétitif (la marche) ou continu (la position allongée sur le lit) (Ulbrecht, Cavanagh et al. 2004), une hygiène faible du pied causant des ulcères à répétition (Connor and Mahdi 2004).

Le développement d'ulcères de pression dépend de la force externe exercée sur la peau et de sa région anatomique. En effet, de nombreux paramètres peuvent varier tels que la géométrie du tissu, les propriétés biomécaniques, la gravité ou l'orientation du corps. On peut distinguer différentes interfaces au niveau desquelles les ulcères peuvent se former. La zone du fessier est composée de l'os pelvique, des muscles glutéaux et de la couche de tissu adipeux sous-cutané.

L'épaisseur des tissus joue un rôle important dans le transfert des charges en position assise. Seules trente minutes suffisent à une compression de 35kPa au niveau du muscle chez le rat pour induire des plaies nécrotiques (Gefen, Gefen et al. 2005). Le support sur lequel le patient est assis a aussi son importance et peut influencer la répartition des forces exercées sur cette zone (Pipkin and Sprigle 2008). La position allongée sur un lit d'hôpital induit des pressions de la peau sur le lit de l'ordre de 55-95 mmHg. Cependant, de faibles pressions sont suffisantes pour générer une occlusion, une hypoxie et une ischémie (Smith 1995). Les propriétés du tissu plantaire dépendent de la posture, de la contraction musculaire et de la zone du pied. Chez des sujets diabétiques, la zone plantaire a été observée plus rigide et plus fine que la normale, surtout au niveau de la tête du premier métatarse (Zheng, Choi et al. 2000; Gefen, Megido-Ravid et al. 2001).

Plusieurs études ont été mises en oeuvre afin de proposer des modèles non-humains d'ulcère de pression.

Parmi ces études on peut citer, à titre d'exemple, des modèles non-humains d'ulcère de pression invasifs tes que ceux décrits dans Peirce, S. M., T. C. Skalak, et al. (2000). "Ischemia-reperfusion injury in chronic pressure ulcer formation: a skin model in the rat." Wound Repair Regen 8(1): 68-76. Dans ce modèle, une plaque d'acier est insérée sous la peau, puis des cycles de compression à l'aide d'un aimant (4 cm de longueur, 2,25 cm de largeur, 1 cm d'épaisseur présentant une pression de 50 mm Hg) permettent d'obtenir une plaie. L'inconvénient de ce type de modèle est qu'il est invasif : il nécessite une incision pour mettre en place la plaque d'acier sous la peau. Il provoque ainsi une inflammation et un processus de cicatrisation au niveau de la zone incisée pouvant interférer avec les phénomènes de cicatrisation de la zone étudiée. Aussi, il isole la peau des tissus sous-jacents. La zone de la plaie à étudier ne bénéficie pas de la chaleur sous-jacente ni de la circulation sanguine.

Un autre modèle non-humains d'ulcère de pression invasif est également décrit dans Reid, R. R., A. C. Sull, et al. (2004), "A novel murine model of cyclical cutaneous ischemia-reperfusion injury." J Surg Res 116(1): 172-180. Dans ce modèle, un anneau magnétique est implanté sous la peau et des cycles de compression à l'aide d'un aimant sur la peau permettent d'obtenir un ulcère de pression. Ce modèle permet d'étudier l'ulcération liée à l'absence de flux sanguin sans compression du tissu au niveau du centre des aimants.

On peut également citer des modèles non-humains d'ulcère de pression non-invasifs, tels que celui décrit dans Saito, Y., M. Hasegawa, et al. (2008). "The Loss of MCP-1 Attenuates Cutaneous Ischemia-Reperfusion Injury in a Mouse Model of Pressure Ulcer." Journal of Investigative Dermatology 128(7): 1838-1851. Dans ce modèle, des cycles de compression à l'aide d'aimants présentant un diamètre de 12 mm, une épaisseur de 5 mm et une pression de 50 mmHg ont été réalisés sur des souris qui ne présentaient pas de diabète. Or, la Demanderesse s'est rendue que le fait d'effectuer des cycles de compression avec une telle pression chez une souris diabétique, ne permettait pas l'obtention d'ulcères de pression.

Demiot, C., V. Sarrazy, et al. (2011), dans "Erythropoietin Restores C-Fiber Function and Prevents Pressure Ulcer Formation in Diabetic Mice." Journal of Investigative Dermatology 131(11): 2316-2322, décrit un modèle d'ulcère de pression chez une souris diabétique neuropathique dans lequel des cycles de compression sont administrés à l'aide d'aimants présentant un diamètre de 10 mm, une épaisseur d'1 mm et une pression de 2000mmHg. Cette force de pression est très importante et peut induire une plaie allant au-delà d'une plaie obtenue de manière physiologique lors de la formation d'un ulcère de pression. A titre d'exemple, la position allongée, pour l'Homme, sur un lit d'hôpital induit des pressions de la peau sur le lit de l'ordre de 55-95 mmHg, elle est de l'ordre de 150 à 500 mmHg sur les ischio en position assise, ce qui est bien en deçà des valeurs de pression appliquées dans ce modèle.

Ainsi, il n'est pas à exclure que dans ce type de modèle, la peau du sujet ne soit altérée au-delà de l'ulcère de pression et que d'autres facteurs physiologiques que ceux inhérents à l'ulcère de pression n'entrent enjeu.

Des pressions similaires sont appliquées à des souris par Danigo, A., L. Magy, et al. (2014). "Neuroprotective Effect of Erythropoietin against Pressure Ulcer in a Mouse Model of Small Fiber Neuropathy." PLoS ONE 9(11): e113454. Dans cette publication, les souris présentent une neuropathie périphérique légère, mais sans diabète.

A l'heure actuelle, il n'existe pas de modèle non-humain d'ulcère de pression induit expérimentalement qui soit reproductible, non invasif et qui permette d'évaluer un traitement sur l'ulcère de pression, pour les sujets diabétiques, en particulier, ceux présentant une neuropathie périphérique.

Ainsi, la présente invention permet d'obtenir des ulcères de pression chez le sujet sain, comme chez le sujet diabétique, en particulier le sujet diabétique présentant une neuropathie périphérique, de manière induite (c'est-à-dire qui se développe après plusieurs jours contrairement à une excision ou une compression forte et prolongée générant l'apparition immédiate d'une plaie), non-invasive (afin de ne pas créer de phénomène d'inflammation qui pourrait interférer avec le processus de cicatrisation de l'ulcère) reproductible, dans un délai court, sur un animal de petite taille (comme la souris) et donc à un moindre coût. Selon un mode préféré de réalisation, la présente invention permet d'obtenir des ulcères de pression chez le sujet sain et chez le sujet diabétique de type I. Ce modèle permet d'obtenir des ulcères de pression de grade 3 (toutes les couches de la peau sont atteintes : hypoderme, derme et épiderme).

Le diabète de type I se caractérise également par une perte de poids importante, ce qui diminue l'épaisseur de la peau et notamment de l'hypoderme. Au contraire, un sujet atteint de diabète de type II est obèse, ce qui a pour conséquence d'augmenter l'épaisseur de la peau et de l'hypoderme du sujet. Il en résulte qu'il est plus facile d'induire des ulcères, en particulier des ulcères de grade 3 chez un sujet diabétique de type II par rapport à un sujet diabétique de type I.

Ce modèle permet également d'obtenir des ulcères de pression sur le sujet non-diabétique, ce qui permet de comparer les paramètres physiologiques du sujet sain versus sujet diabétique. Ce modèle permet un suivi du processus de cicatrisation et peut être utilisé pour tester des méthodes de traitement, des pansements ou des substances actives afin d'évaluer leur efficacité.

Ce fascicule utilise l'unité mmHg. Cette dernière peut être convertie en l'unité SI Pa sur la base du facteur de conversion 1 mmHg = 133,322 Pa.

La présente invention porte donc sur un modèle d'ulcère de pression sur un rongeur sur lequel les étapes suivantes sont mises en oeuvre :
a) l'application d'une compression magnétique perpendiculaire à la surface de la peau dudit mammifère à l'aide de deux aimants de part et d'autre d'un pli de peau dudit rongeur
b) le retrait des aimants
c) la pose d'un pansement à l'endroit des aimants suivi d'une friction à l'endroit des aimants caractérisée par une valeur de pression appliquée sur les zones où les aimants sont appliqués comprise entre 49 et 147 mm Hg, de préférence comprise entre 73 et 123 mm Hg,
d) l'application d'une seconde compression magnétique perpendiculaire à la surface de la peau à l'aide de deux aimants de part et d'autre d'un pli de peau dudit rongeur
e) le retrait des aimants suivi d'une friction à l'endroit des aimants
f) la répétition des étapes d) et e) au moins une fois, de préférence au moins deux
g) le retrait du pansement,
dans lequel les aimants utilisés présentent de préférence une pression de 50 à 700mmHg ; et dans lequel la durée d'application des aimants à l'étape a) et d) est d'au moins 2 heures.

L'invention a également pour objet l'utilisation d'un tel modèle d'ulcère de pression pour l'évaluation de l'efficacité d'une substance active et/ou d'un dispositif pour la cicatrisation de plaies sur lesquelles une substance active a été administrée et/ou un dispositif a été apposé.

Selon un autre mode de réalisation, la présente invention a également pour objet un procédé d'évaluation d'un traitement de cicatrisation d'une plaie chez un rongeur sain ou diabétique, induite au moyen d'un modèle tel que décrit précédemment, comprenant :
a) l'application d'au moins une substance active et/ou dispositif sur ladite plaie; et
b) la caractérisation de l'effet du traitement sur la cicatrisation de ladite plaie.

Enfin, selon un autre mode de réalisation, la présente invention a également pour objet un procédé d'évaluation d'un traitement de cicatrisation d'une plaie chez un rongeur sain ou diabétique, induite au moyen d'un modèle tel que défini précédemment sur laquelle une substance active a été administrée et/ou un dispositif a été apposé, comprenant :
a) la caractérisation de l'effet du traitement sur la cicatrisation de ladite plaie.

Selon un autre mode de réalisation, l'invention a aussi pour objet un procédé d'évaluation d'un traitement de cicatrisation d'ulcères de pression chez un rongeur diabétique comprenant :
a) l'application d'une compression magnétique perpendiculaire à la surface de la peau dudit mammifère à l'aide de deux aimants de part et d'autre d'un pli de peau dudit rongeur
b) le retrait des aimants
c) la pose d'un pansement à l'endroit des aimants suivi d'une friction à l'endroit des aimants caractérisée par une valeur de pression appliquée sur les zones où les aimants sont appliqués comprise entre 49 et 147 mm Hg, de préférence comprise entre 73 et 123 mm Hg,
d) l'application d'une seconde compression magnétique perpendiculaire à la surface de la peau à l'aide de deux aimants de part et d'autre d'un pli de peau dudit rongeur
e) le retrait des aimants suivi d'une friction à l'endroit des aimants
f) la répétition des étapes d) et e) au moins une fois, de préférence au moins deux
g) le retrait du pansement,
h) dans lequel les aimants utilisés présentent de préférence une pression de 50 à 700mmHg ; et dans lequel la durée d'application des aimants à l'étape a) et d) est d'au moins 2 heures.

Le rongeur utilisé dans le cadre de la présente invention est de préférence une souris ou un rat, de préférence une souris, et encore plus préférentiellement une souris de laboratoire.

Les rongeurs sont classiquement préférés en laboratoire pour leur prix plus abordables et leur nombre plus importants. Le cochon peut aussi être utilisé, cependant la peau du cochon est bien plus épaisse que celle de la souris (1500µm versus 200µm) [Wei et al, 2017], il est donc généralement difficile de transposer un protocole effectué sur une souris sur un cochon et inversement.

Les aimants utilisés dans le cadre de la présente invention présentent une pression de 50 à 700mmHg, de préférence 200 à 600, préférentiellement entre 400 et 550 mmHg. La taille des aimants est choisie de sorte à laisser un espace d'au moins 5 mm de peau non compressée entre les aimants. Par exemple, dans le cadre de la présente invention, lorsque le rongeur est une souris, l'aimant présente un diamètre de 6 à 8 mm.

La friction mise en oeuvre à l'étape c) est, de préférence une friction appuyée, pouvant être réalisée à l'aide de tout ustensile permettant d'exercer une friction, de préférence, à l'aide du doigt ou d'un bâtonnet ouaté. Cette friction, de manière optimale, consiste en au moins une friction appuyée, de préférence au moins 1 aller et 1 retour de haut en bas puis 1 aller et 1 retour de gauche à droite des zones où les aimants sont appliqués. Cette friction se fait sur le pansement mis en place à l'étape c).

La friction mise en oeuvre à l'étape c) est aussi caractérisée par une valeur de pression appliquée sur les zones où les aimants sont appliqués comprise entre 49 et 147 millimètre de mercure (mm Hg), de préférence comprise entre 73 et 123 mm Hg.

Le pansement est, de préférence, composé d'un film mince adhésivé. A titre d'exemple, on peut citer un film de polyuréthane adhésivé tel que le produit Optiskin Film^{®} commercialisé par les Laboratoires URGO.

Les inventeurs ont découvert de façon surprenante que l'application d'un pansement selon l'invention permet avantageusement d'obtenir un modèle d'ulcère de pression viable et reproductible que le sujet soit diabétique, en particulier présentant un diabète de type I, ou sain. En effet, il est apparu que l'application d'un pansement favorisait la macération de la plaie et permettait une reproduction plus fiable d'un ulcère de pression. De manière préférentielle, si nécessaire, la peau du rongeur sera rasée ou dépilée avant l'étape a), de préférence dépilée.

En particulier, la durée d'application des aimants à l'étape a) et d) est d'au moins 2 heures, de préférence au moins 3 heures. Dans un mode préférentiel de l'invention cette durée d'application ne dépassera pas 8 heures, de préférence 6 heures.

De préférence, l'étape g) de retrait du pansement est réalisée au moins 8 heures après la fin de l'étape e) de retrait des aimants suivi d'une friction appuyée.

L'étape f) de répétition des étapes d) et e) est espacée d'au moins 15 heures, de préférence d'au moins 18 heures, c'est-à-dire qu'entre chaque répétition de l'étape e), on laisse la peau dudit rongeur au repos pendant au moins 15 heures, de préférence au moins 18 heures.

Ces différentes étapes mises en oeuvre pour réaliser le modèle selon l'invention permettent d'aboutir au développement progressif d'un ulcère cutané de grade III, et ce quel que soit le statut sain ou diabétique (neuropathique ou neuro ischémique) du rongeur étudié.

La présente invention a également pour objet un procédé d'évaluation d'un traitement de cicatrisation d'ulcères de pression chez un rongeur diabétique, induits au moyen d'un modèle tel que décrit précédemment, comprenant :
a) l'application d'au moins une substance active et/ou dispositif sur une plaie présente sur ledit modèle; et
b) la caractérisation de l'effet du traitement sur la cicatrisation de ladite plaie.

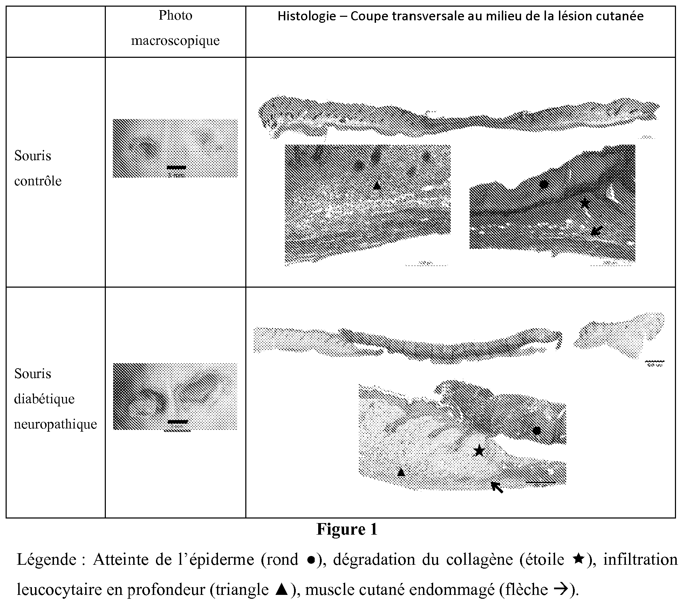

Dans le cadre de ce procédé, la plaie a été induite sur le modèle d'ulcère de pression précédemment décrit. En particulier, la plaie a été induite sur les zones où les aimants ont été apposés
La figure 1 est une photographie d'ulcère de pression obtenu selon l'invention chez la souris contrôle et chez la souris diabétique, ainsi qu'une analyse histologique de la peau montrant une atteinte de toutes les couches de la peau.

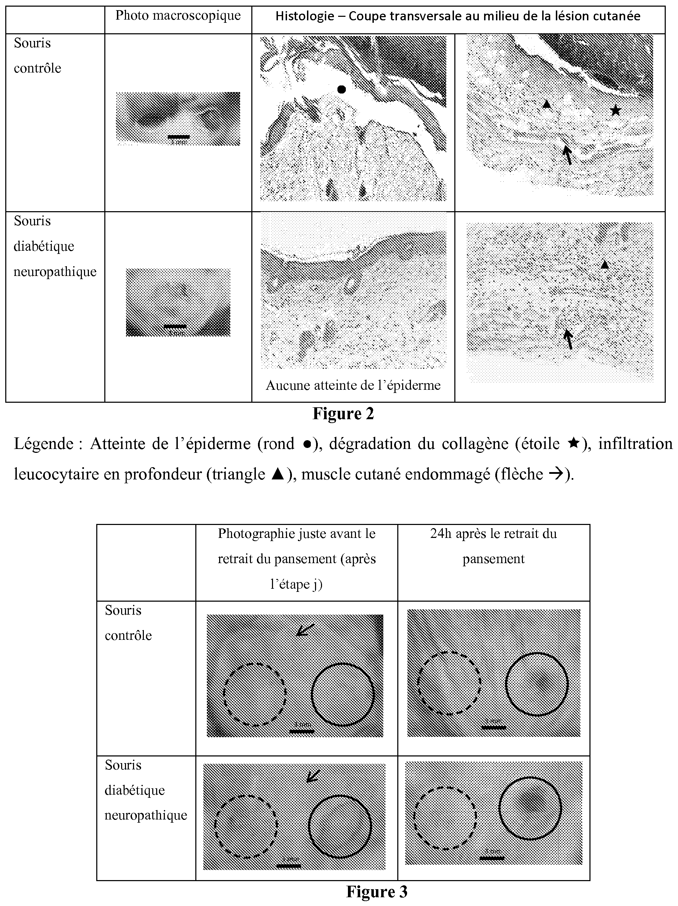

La figure 2 est une analyse histologique de l'hypoderme, derme et épiderme de la souris contrôle et la souris diabétique lorsque la souris n'est soumise ni à une friction appuyée (, ni à la pose d'un pansement après la compression magnétique (mises en oeuvre au cours de l'étape c) selon l'invention), ni au retrait dudit pansement (étape g) selon l'invention), illustrée à l'exemple 2 comparatif.
La figure 3 est une photographie des lésions induites après la compression de la peau entre aimants avec pose d'un pansement, et avec friction sur la zone de droite (cercle en trait continu) mais pas la zone de gauche (cercle en trait discontinu), illustrée à l'exemple 3 comparatif. La lésion de type ulcère n'apparait que lorsqu'il y a compression + pansement + friction.

### Exemples :

Des souris C57BI6 (Janvier, Saint Genest Saint Isle, France) pesant environ 23g sont divisées en deux groupes : un groupe contrôle et un groupe diabétique. Un diabète est induit chez la souris en faisant une seule injection de streptozotocine à 180 mg/kg par voie intra-péritonéale.

Le streptozotocine est préalablement dilué dans du tampon citrate. L'injection est faite sous anesthésie isoflurane. La glycémie des souris est alors mesurée tous les jours pendant 4 jours afin de vérifier si le diabète a bien été induit.

### Exemple 1 de modèle selon l'invention :

66 souris diabétiques et 58 souris contrôles ont été dépilées avec une crème dépilatoire Veet^{®} (Reckitt Benckisser), puis soumises à des cycles de pression selon l'invention :
a) l'application d'une compression magnétique perpendiculaire à la surface de la peau à l'aide de deux aimants présentant un diamètre de 8 mm et une pression de 418 mmHg de part et d'autre d'un pli de peau pendant 3 heures
b) le retrait des aimants
c) la pose d'un pansement Optiskin Film^{®} des Laboratoires URGO à l'endroit des aimants suivie d'une friction appuyée à l'aide un bâtonnet ouaté (5 allers et 5 retours de haut en bas puis 5 allers et 5 retours de gauche à droite) à l'endroit des aimants
d) l'application d'une seconde compression magnétique perpendiculaire à la surface de la peau à l'aide des deux mêmes aimants de part et d'autre du pli de peau 21 heures après le retrait des aimants de l'étape b) pendant 3 heures
e) le retrait des aimants suivi d'une friction appuyée à l'aide un bâtonnet ouaté (5 allers et 5 retours de haut en bas puis 5 allers et 5 retours de gauche à droite) à l'endroit des aimants
f) l'application d'une troisième compression magnétique perpendiculaire à la surface de la peau à l'aide des deux mêmes aimants de part et d'autre du pli de peau 21 heures après le retrait des aimants de l'étape b) pendant 3 heures
g) le retrait des aimants suivi d'une friction appuyée à l'aide un bâtonnet ouaté (5 allers et 5 retours de haut en bas puis 5 allers et 5 retours de gauche à droite) à l'endroit des aimants
h) le retrait du pansement

L'étape c) de friction a été réalisée par différentes personnes afin de vérifier si le geste n'était pas personne-dépendant.

24 heures après le retrait du pansement, la lésion est maximale. On observe alors un ulcère de pression de stade III chez la souris contrôle et chez la souris diabétique illustrée à la figure 1.

### Exemple 2 de modèle comparatif :

13 souris diabétiques et 13 souris contrôles ont été dépilées avec une crème dépilatoire Veet^{®} (Reckitt Benckisser), puis soumises à des cycles de pression:
a) l'application d'une compression magnétique perpendiculaire à la surface de la peau à l'aide de deux aimants présentant un diamètre de 8 mm et une pression de 418 mmHg de part et d'autre d'un pli de peau pendant 3 heures
b) le retrait des aimants
c) l'application d'une compression magnétique perpendiculaire à la surface de la peau à l'aide des deux mêmes aimants de part et d'autre du pli de peau 21 heures après le retrait des aimants de l'étape b) pendant 3 heures
d) le retrait des aimants
e) l'application d'une compression magnétique perpendiculaire à la surface de la peau à l'aide des deux mêmes aimants de part et d'autre du pli de peau 21 heures après le retrait des aimants de l'étape b) pendant 3 heures
f) le retrait des aimants

Comme illustrée à la figure 2, sans frottements ni pansements, seules les souris contrôles développaient des ulcères visibles à l'oeil nu avec (d'après l'analyse histologique) atteinte des 3 couches de la peau (hypoderme, derme et épiderme). Les souris diabétiques neuropathiques n'avaient pas d'atteinte visible à l'oeil nu et l'histologie a montré une atteinte uniquement sur l'hypoderme et le derme profond (pas l'épiderme).

### Exemple 3 de modèle comparatif :

5 souris diabétiques et 10 souris contrôles ont été dépilées avec une crème dépilatoire Veet^{®} (Reckitt Benckisser), puis soumises à des cycles de pression:
a) l'application d'une compression magnétique perpendiculaire à la surface de la peau à l'aide de deux aimants présentant un diamètre de 8 mm et une pression de 418 mmHg de part et d'autre d'un pli de peau pendant 3 heures
b) le retrait des aimants
c) la pose d'un pansement Optiskin Film^{®} des Laboratoires URGO à l'endroit des aimants
d) l'application à l'endroit de l'aimant de droite, d'une friction appuyée à l'aide un bâtonnet ouaté (5 allers et 5 retours de haut en bas puis 5 allers et 5 retours de gauche à droite) ; et aucune friction à l'endroit de l'aimant de gauche
e) l'application d'une compression magnétique perpendiculaire à la surface de la peau à l'aide des deux mêmes aimants de part et d'autre du pli de peau 21 heures après le retrait des aimants de l'étape b) pendant 3 heures
f) le retrait des aimants
g) l'application à l'endroit de l'aimant de droite, d'une friction appuyée à l'aide d'un bâtonnet ouaté (5 allers et 5 retours de haut en bas puis 5 allers et 5 retours de gauche à droite) ; et aucune friction à l'endroit de l'aimant de gauche
h) l'application d'une compression magnétique perpendiculaire à la surface de la peau à l'aide des deux mêmes aimants de part et d'autre du pli de peau 21 heures après le retrait des aimants de l'étape f) pendant 3 heures
i) le retrait des aimants
j) l'application à l'endroit de l'aimant de droite, d'une friction appuyée à l'aide un bâtonnet ouaté (5 allers et 5 retours de haut en bas puis 5 allers et 5 retours de gauche à droite) ; et aucune friction à l'endroit de l'aimant de gauche
k) le retrait du pansement

Comme illustrée à la figure 3, sans frottements mais avec application d'un pansement (zone de gauche sur l'animal), ni les souris contrôles, ni les souris diabétiques neuropathiques n'ont développé d'ulcère de grade III. Un érythème léger sur certains animaux a pu être observé. L'histologie a montré une atteinte sur l'hypoderme et le derme profond chez la souris contrôle et la souris diabétique neuropathique.

Le pansement permet donc une aggravation de la plaie mais dans un degré moindre que le frottement seul, et de manière non reproductible entre animaux.

## Revendications

1. Modèle d'ulcère de pression sur un rongeur sur lequel les étapes suivantes sont mises en oeuvre :
a) l'application d'une compression magnétique perpendiculaire à la surface de la peau dudit rongeur à l'aide de deux aimants de part et d'autre d'un pli de peau dudit rongeur
b) le retrait des aimants
c) la pose d'un pansement à l'endroit des aimants suivi d'une friction à l'endroit des aimants **caractérisée par** une valeur de pression appliquée sur les zones où les aimants sont appliqués comprise entre 6532,8 et 19598,4 Pa (49 et 147 mmHg) de préférence comprise entre 9732,5 et 16398,7 Pa (73 et 123 mmHg),
d) l'application d'une seconde compression magnétique perpendiculaire à la surface de la peau à l'aide de deux aimants de part et d'autre d'un pli de peau dudit rongeur
e) le retrait des aimants suivi d'une friction à l'endroit des aimants,
f) la répétition des étapes d) et e) au moins une fois, de préférence au moins deux fois
g) le retrait du pansement,
dans lequel les aimants utilisés présentent de préférence une pression de 6666,1 à 93325,7 Pa (50 à 700 mmHg) et dans lequel la durée d'application des aimants à l'étape a) et d) est d'au moins 2 heures.

2. Modèle selon la revendication 1, dans lequel le rongeur est une souris.

3. Modèle selon la revendication 1 ou 2, dans lequel le rongeur est un sujet sain ou diabétique, de préférence diabétique de type I.

4. Modèle selon l'une quelconque des revendications précédentes, dans lequel les aimants utilisés présentent de préférence une pression de 26664,5 à 79993,4 Pa (200 à 600 mmHg), préférentiellement entre 53329 et 73327,3 Pa (400 et 550 mmHg).

5. Modèle selon l'une quelconque des revendications précédentes, dans lequel la friction opérée à l'étape c) est une friction appuyée, par exemple réalisée à l'aide d'un bâtonnet ouaté.

6. Modèle selon l'une quelconque des revendications précédentes, dans lequel le pansement est un film mince adhésivé.

7. Modèle selon l'une quelconque des revendications précédentes, dans lequel la peau dudit rongeur est rasée ou dépilée avant l'étape a), de préférence dépilée.

8. Modèle selon l'une quelconque des revendications précédentes, dans lequel la durée d'application des aimants à l'étape a) et d) est d'au moins 3 heures.

9. Modèle selon l'une quelconque des revendications précédentes, dans lequel la durée d'application des aimants à l'étape a) et d) ne dépasse pas 8 heures, de préférence 6 heures.

10. Modèle selon l'une quelconque des revendications précédentes, dans lequel le retrait du pansement à l'étape g) est réalisé au moins 8 heures après la fin de l'étape e) de retrait des aimants suivi d'une friction appuyée.

11. Modèle selon l'une quelconque des revendications précédentes, dans lequel l'étape f) de répétition des étapes d) et e) est espacée d'au moins 15 heures, de préférence d'au moins 18 heures.

12. Utilisation d'un modèle d'ulcère de pression selon l'une quelconque des revendications précédentes, pour l'évaluation de l'efficacité d'une substance active et/ou d'un dispositif pour la cicatrisation de plaies sur lesquelles ladite substance active a été administrée et/ou ledit dispositif a été apposé.

13. Procédé d'évaluation d'un traitement de cicatrisation d'une plaie chez un rongeur sain ou diabétique, de préférence diabétique de type I, induite au moyen d'un modèle selon l'une quelconque des revendications 1 à 11, sur laquelle une substance active a été administrée et/ou un dispositif a été apposé, comprenant la caractérisation de l'effet du traitement sur la cicatrisation de ladite plaie.

14. Procédé selon la revendication 13, dans lequel le rongeur est une souris.

## Patentansprüche

1. Modell eines Druckgeschwürs bei einem Nagetier, bei dem die folgenden Schritte durchgeführt werden:
a) Anlegen einer magnetischen Kompression senkrecht zur Hautoberfläche des Nagetiers mithilfe von zwei Magneten auf beiden Seiten einer Hautfalte des Nagetiers,
b) Entfernen der Magnete,
c) Anbringen eines Verbands an der Stelle der Magnete, gefolgt von einer Reibung an der Stelle der Magnete, **gekennzeichnet durch** einen Druckwert, der auf die Bereiche, an denen die Magnete angebracht sind, ausgeübt wird, zwischen 6532,8 und 19598,4 Pa (49 und 147 mmHg), bevorzugt zwischen 9732,5 und 16398,7 Pa (73 und 123 mmHg),
d) Anlegen einer zweiten magnetischen Kompression senkrecht zur Hautoberfläche mithilfe von zwei Magneten auf beiden Seiten einer Hautfalte des Nagetiers,
e) Entfernen der Magnete, gefolgt von einer Reibung an der Stelle der Magnete,
f) Wiederholen der Schritte d) und e) wenigstens einmal, bevorzugt wenigstens zweimal,
g) Entfernen des Verbands,
wobei die verwendeten Magnete bevorzugt einen Druck von 6666,1 bis 93325,7 Pa (50 bis 700 mmHg) aufweisen und wobei die Dauer des Anlegens der Magnete in Schritt a) und d) wenigstens 2 Stunden beträgt.

2. Modell nach Anspruch 1, wobei es sich bei dem Nagetier um eine Maus handelt.

3. Modell nach Anspruch 1 oder 2, wobei das Nagetier ein gesundes Tier oder ein Tier mit Diabetes ist, bevorzugt mit Typ-1-Diabetes.

4. Modell nach einem der vorhergehenden Ansprüche, wobei die verwendeten Magnete bevorzugt einen Druck von 26664,5 bis 79993,4 Pa (200 bis 600 mmHg), bevorzugt zwischen 53329 und 73327,3 Pa (400 und 550 mmHg), aufweisen.

5. Modell nach einem der vorhergehenden Ansprüche, wobei die in Schritt c) vorgenommene Reibung eine Druckreibung ist, die z. B. mit einem Wattestäbchen durchgeführt wird.

6. Modell nach einem der vorhergehenden Ansprüche, wobei der Verband ein dünner Klebefilm ist.

7. Modell nach einem der vorhergehenden Ansprüche, wobei die Haut des Nagetiers vor Schritt a) rasiert oder enthaart, bevorzugt enthaart wird.

8. Modell nach einem der vorhergehenden Ansprüche, wobei die Dauer der Anwendung der Magnete in Schritt a) und d) wenigstens 3 Stunden beträgt.

9. Modell nach einem der vorhergehenden Ansprüche, wobei die Dauer der Anwendung der Magnete in Schritt a) und d) nicht mehr als 8 Stunden, bevorzugt 6 Stunden, beträgt.

10. Modell nach einem der vorhergehenden Ansprüche, wobei das Entfernen des Verbands in Schritt g) wenigstens 8 Stunden nach dem Ende von Schritt e) des Entfernens der Magnete mit anschließender Druckreibung durchgeführt wird.

11. Modell nach einem der vorhergehenden Ansprüche, wobei Schritt f) der Wiederholung der Schritte d) und e) wenigstens 15 Stunden, bevorzugt wenigstens 18 Stunden, auseinander liegt.

12. Verwendung eines Modells eines Druckgeschwürs nach einem der vorhergehenden Ansprüche zur Bewertung der Wirksamkeit eines Wirkstoffs und/oder einer Vorrichtung zur Heilung von Wunden, auf die der Wirkstoff verabreicht und/oder die Vorrichtung aufgebracht wurde.

13. Verfahren zur Bewertung einer Behandlung zur Heilung einer Wunde bei einem gesunden oder diabetischen Nagetier, bevorzugt mit Typ-1-Diabetes, die mittels eines Modells nach einem der Ansprüche 1 bis 11 induziert wurde, auf die ein Wirkstoff verabreicht und/oder eine Vorrichtung aufgebracht wurde, umfassend die Charakterisierung der Wirkung der Behandlung auf die Heilung der Wunde.

14. Verfahren nach Anspruch 13, wobei es sich bei dem Nagetier um eine Maus handelt.

## Claims

1. A model of pressure ulcer on a rodent on which the following steps are implemented:
a) applying a magnetic compression perpendicular to the surface of the skin of said rodent using two magnets on either side of a skin fold of said rodent,
b) removing the magnets,
c) placing a bandage at the location of the magnets followed by friction at the location of the magnets **characterised by** a value of the pressure applied on the areas where the magnets are applied comprised between 6,532.8 and 19,598.4 Pa (49 and 147 mmHg), preferably comprised between 9,732.5 and 16,398.7 Pa (73 and 123 mmHg),
d) applying a second magnetic compression perpendicular to the surface of the skin using two magnets on either side of a skin fold of said rodent,
e) removing the magnets followed by friction at the location of the magnets,
f) repeating steps d) and e) at least once, preferably at least twice,
g) removing the bandage,
wherein the used magnets preferably have a pressure of 6,666.1 to 93,325.7 Pa (50 to 700 mmHg);
and wherein the duration of application of the magnets in step a) and d) is at least 2 hours.

2. The model according to claim 1, wherein the rodent is a mouse.

3. The model according to claim 1 or 2, wherein the rodent is a healthy or diabetic, preferably type I diabetic, subject.

4. The model according to any one of the preceding claims, wherein the used magnets preferably have a pressure of 26,664.5 to 79,993.4 Pa (200 to 600 mmHg), preferably between 53,329 and 73,327.3 Pa (400 and 550 mmHg).

5. The model according to any one of the preceding claims, wherein the friction performed in step c) is friction under pressure, for example carried out using a cotton swab.

6. The model of any one of the preceding claims, wherein the bandage is a thin adhesive film.

7. The model according to any one of the preceding claims, wherein the skin of said rodent is shaved or depilated before step a), preferably depilated.

8. The model according to any one of the preceding claims, wherein the duration of application of the magnets in step a) and d) is at least 3 hours.

9. The model according to any one of the preceding claims, wherein the duration of application of the magnets in step a) and d) does not exceed 8 hours, preferably 6 hours.

10. The model according to any one of the preceding claims, wherein the removal of the bandage in step g) is carried out at least 8 hours after the end of step e) of removing the magnets followed by friction under pressure.

11. The model according to any one of the preceding claims, wherein step f) of repeating steps d) and e) is done with a time interval of at least 15 hours, preferably at least 18 hours.

12. A use of a pressure ulcer model according to any one of the preceding claims, for evaluating the effectiveness of an active substance and/or a device for healing wounds over which said active substance has been administered and/or said device has been placed.

13. A method for evaluating a wound healing treatment in a healthy or diabetic, preferably type I diabetic, rodent, induced by means of a model according to any one of claims 1 to 11, on which an active substance has been administered and/or a device has been placed, comprising characterising the effect of the treatment on healing of said wound.

14. The method according to claim 13, wherein the rodent is a mouse.
